# FASCICULE DE BREVET EUROPEEN

(11) **EP 1 719 481 B1**
(45) Date de publication et mention de la délivrance du brevet: **29.04.2009**
(21) Numéro de dépôt: 05009874.8
(22) Date de dépôt: 06.05.2005
(51) Int. Cl.: A61F 5/56, A61C 7/36

(54) **Dispositif de propulsion mandibulaire**
Vorrichtung zum Strecken des Unterkiefers
Mandibular advancement device

(43) Date de publication de la demande: 08.11.2006
(73) Titulaire: Arni, Pierre, 1213 Petit-Lancy (CH)
(72) Inventeur: Arni, Pierre, 1213 Petit-Lancy (CH)
(74) Mandataire: Micheli & Cie SA

(56) Documents cités:
- EP-A- 1 459 699
- DE-U1- 29 506 512
- FR-A- 2 816 203
- FR-A- 2 831 427
- US-A- 5 378 147
- US-A- 6 012 920

## Description

La présente invention a pour objet un dispositif de propulsion mandibulaire comportant une gouttière dentaire supérieure destinée à être placée sur l'arcade dentaire supérieure d'un patient et une gouttière dentaire inférieure destinée à être placée sur l'arcade dentaire inférieure ainsi que deux liens latéraux adaptés à relier les deux gouttières de manière à avancer l'arcade dentaire inférieure en cas d'un mouvement vertical entre les deux arcades dentaires, chaque lien latéral comportant un moyen de traction attaché par ses extrémités aux gouttières.

En général, ce genre d'appareil dentaire qui est utilisé contre le ronflement voire l'apnée du sommeil est connu dans le domaine de l'orthopédie dento-faciale sous la dénomination de propulseur. Il existe aussi d'autres types d'appareils utilisés dans ce contexte, mais pour ce qui suit uniquement les dispositifs intra-oraux et spécifiquement du type mentionné ci-dessus sont importants. En ce qui concerne en particulier le phénomène du ronflement, celui-ci est normalement causé par un faible tonus de la musculature du cou pendant le sommeil. Les voies respiratoires sont ainsi resserrées voire même obturées en cas d'apnée. La circulation d'air dans les voies respiratoires resserrées s'effectue alors à une vitesse plus élevée que normalement et génère du bruit en mettant en mouvement les structures relâchées dans le cou. De ce fait le phénomène est plus fréquent chez les personnes âgées dont la musculature a tendance à perdre du tonus avec l'âge.

Les appareils de ce genre, comme par exemple décrit dans FR 2 816 203, tendent à rétablir une circulation d'air normale, surtout à une vitesse normale, en essayant d'élargir les voies respiratoires. A cet effet, ils maintiennent en règle générale le maxillaire inférieur en avant afin d'ouvrir la partie pharyngale des voies respiratoires, ceci par l'intermédiaire des liens latéraux ayant une longueur adéquate et raccordant les gouttières dentaires supérieure et inférieure habituellement placées pendant la nuit sur les arcades dentaires supérieure et inférieure.

En particulier, les liens latéraux des appareils connus subissent une traction dans le cas d'un mouvement vertical entre les maxillaires supérieur et inférieur, c'est-à-dire quand la respiration s'effectue par la bouche ouverte. Les liens sont par conséquent normalement en un matériau flexible, mais ne permettent pas une extension longitudinale des liens. En effet, en orientant l'axe longitudinal des liens montés sur le coté extérieur des gouttières dans un sens allant de l'arrière bas du maxillaire inférieur à l'avant haut du maxillaire supérieur, une ouverture de la bouche due à un abaissement du maxillaire inférieur pendant le sommeil provoque ainsi une propulsion du maxillaire inférieur en avant et une ouverture correspondante des voies respiratoires. Le maxillaire inférieur, et avec lui la base de la langue, est alors maintenu voire propulsé en avant afin de contribuer à laisser la partie pharyngale des voies respiratoires suffisamment ouverte pour établir ou favoriser une respiration normale sans ronflement voire dans certains cas pour diminuer les apnées du sommeil.

Les liens latéraux sont conventionnellement fixés sur les gouttières, par exemple par clipsage, ce qui entraîne plusieurs inconvénients. D'abord, le clipsage est effectué sur le coté extérieur d'une gouttière aux endroits où le lien doit être monté. La joue du patient et l'intérieur de sa bouche sont alors directement exposés à l'extrémité du lien voire éventuellement à une pièce supplémentaire nécessitée par le clipsage, ce qui peut provoquer un sentiment d'inconfort ou un frottement voire une blessure en fonction de la taille et de la forme de ces pièces, ce qui peut amener le patient à effectivement ne plus utiliser le dispositif. Au niveau de la fonctionnalité du dispositif, un clipsage n'est pas la solution idéale parce qu'il est souhaitable que les liens disposent d'une certaine mobilité latérale et rotative afin de garantir une mobilité correspondante notamment latérale et verticale entre les maxillaires supérieur et inférieur. En même temps, une telle articulation doit être suffisamment résistante pour éviter la cassure. De plus, si les liens sont montés définitivement par clipsage, l'utilisateur est privé de la possibilité d'adapter la longueur des liens latéraux lui-même en les changeant. Dans ce cas, un clipsage complique aussi la fabrication d'un tel dispositif et implique des coûts respectifs pour le fabricant.

Le but de la présente invention est de remédier à ces inconvénients et notamment de proposer un dispositif permettant la protection de l'intérieur de la bouche du patient, tout en disposant d'une articulation moins fragile ainsi que permettant une mobilité améliorée des extrémités des liens et en facilitant la fabrication et l'utilisation du dispositif, en particulier pour permettre le changement des liens par l'utilisateur lui-même.

L'invention est caractérisée à cet effet par les caractéristiques énumérées aux revendications 1, 2 et 10.

Un dispositif selon la présente invention comprend notamment des liens latéraux ayant des rotules avec une ouverture adaptée à recevoir une extrémité d'un moyen de traction du lien, chaque gouttière du dispositif comprenant deux logements latéraux adaptés à héberger une rotule connectée à un moyen de traction. Les liens latéraux sont ainsi connectés aux gouttières par l'intermédiaire d'un joint à rotule. De plus, les extrémités des liens se trouvent à l'intérieur de logements latéraux qui ont une surface extérieure lisse et forment de ce fait une protection pour la bouche du patient. Il est à noter également que la taille est un facteur important pour ce genre de dispositifs et qu'un tel lien est particulièrement adapté à être miniaturisé et ainsi à réduire l'encombrement de cet appareil dentaire afin de permettre un confort amélioré pour le patient.

En particulier, un lien latéral destiné à être intégré dans un tel dispositif de propulsion mandibulaire peut être fabriqué indépendamment de ce dispositif et se distingue par le fait que les extrémités du moyen de traction du lien sont adaptées à être logées de façon amovible dans une ouverture d'une rotule du lien latéral, cette rotule connectée à un moyen de traction étant adaptée à être hébergée de façon amovible dans un logement latéral d'une gouttière, de manière à ce que le lien latéral puisse être connecté aux gouttières de façon amovible par l'intermédiaire d'un joint à rotule.

Le procédé d'assemblage d'un tel dispositif de propulsion mandibulaire comporte les étapes de faire passer une extrémité d'un moyen de traction d'un lien latéral à travers un trou pratiqué dans le logement latéral positionné dans l'axe longitudinal du moyen de traction, d'emboîter une rotule du lien latéral sur l'extrémité du moyen de traction traversant le trou du logement latéral, d'encliqueter cette rotule dans une alvéole intérieure du logement latéral et de répéter ces étapes pour toutes les extrémités des moyens de traction des deux liens latéraux du dispositif, de manière à ce que les liens latéraux soient connectés latéralement aux gouttières par l'intermédiaire d'un joint à rotule. L'assemblage et la fabrication ainsi que l'utilisation du dispositif sont alors facilités, en particulier pour le changement des liens par l'utilisateur. En effet, celui-ci peut lui-même effectuer manuellement cette dernière opération sans aucun problème du fait que chaque articulation est réalisée par emboîtement de trois pièces qui ne sont pas montées l'une sur l'autre de manière définitive. Simultanément, une telle articulation est très résistante contre une cassure parce que l'extrémité d'un lien est logée de façon mobile dans les alvéoles des gouttières et parce que la force de traction sur les liens s'applique contre les parois de ces alvéoles au lieu de s'appliquer à des pièces fragiles utilisées pour un clipsage comme dans l'art antérieur.

D'autres avantages ressortent des caractéristiques exprimées dans les revendications dépendantes et de la description exposant ci-après l'invention plus en détail à l'aide des dessins.

Les dessins annexés représentent, à titre d'exemple, une forme d'exécution de l'invention.

La figure 1a est une vue en perspective schématique d'un dispositif selon la présente invention montrant un lien latéral monté sur des gouttières dentaires.

La figure 1b illustre schématiquement une coupe horizontale à travers le dispositif de la figure 1 a au niveau des logements latéraux, une rotule connectée à l'extrémité d'un moyen de traction d'un lien latéral qui y est logée.

La figure 1c représente une vue partielle de la figure 1b, montrant en détail un logement latéral avec une rotule connectée à une extrémité d'un moyen de traction d'un lien latéral.

La figure 2a est une vue en perspective d'un lien latéral avec un moyen de traction et des rotules correspondantes.

La figure 2b montre une vue de coté des pièces de la figure 2a.

La figure 2c est une vue de dessous des pièces de la figure 2a, une fois assemblées.

L'invention va maintenant être décrite en détail en référence aux dessins annexés illustrant à titre d'exemple une forme d'exécution de l'invention.

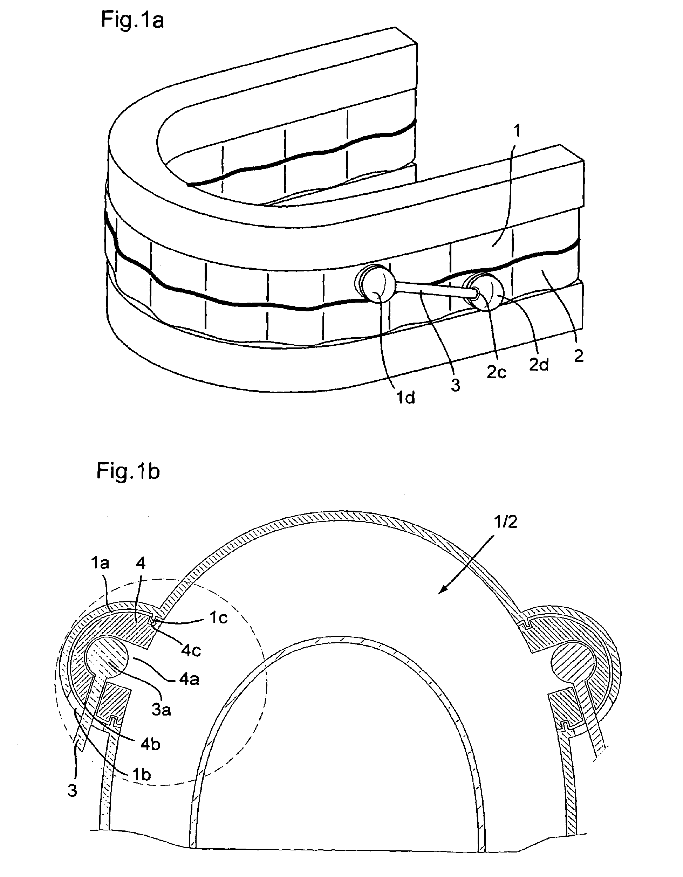

En se référant à la figure 1a, les composants importants d'un dispositif de propulsion mandibulaire selon la présente invention sont clairement visibles. Ils comportent une gouttière dentaire supérieure 1 destinée à être placée sur l'arcade dentaire supérieure d'un patient et une gouttière dentaire inférieure 2 destinée à être placée sur l'arcade dentaire inférieure. Les deux gouttières dentaires 1 et 2 sont normalement fabriquées par thermoformage en utilisant par exemple une empreinte des dents du patient afin de reproduire exactement le profil individuel de son arcade dentaire correspondante sur laquelle elles peuvent ensuite être fixées par encliquetage, surtout pendant la nuit. Les gouttières dentaires sont, de préférence, en un matériau plastique transparente thermoformable, par exemple connu sous les noms commerciaux Duran ou Essix, mais elles peuvent être fabriquées par un autre procédé que le thermoformage ainsi qu'en tout autre matériau adapté. Les modes de fabrication conventionnels de ces gouttières peuvent en effet être utilisés, avec une étape supplémentaire décrite en détail plus loin dans le texte, également pour les dispositifs selon la présente invention.

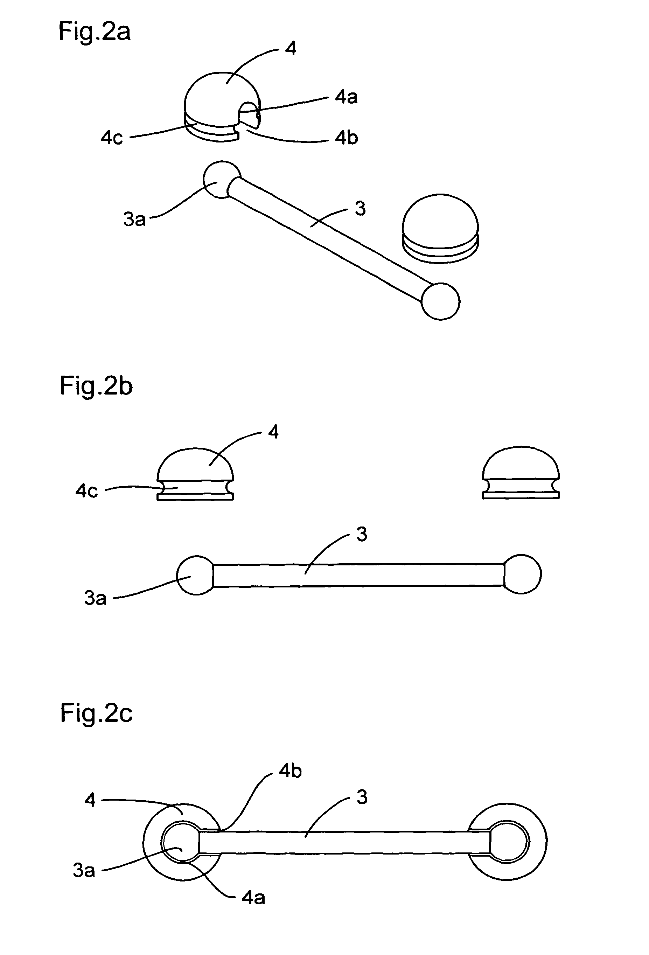

Le dispositif comporte encore deux liens latéraux adaptés à relier les deux gouttières 1, 2 de manière à avancer l'arcade dentaire inférieure en cas d'un mouvement vertical entre les deux arcades dentaires. Comme illustré aux figures 2a à 2c, chaque lien latéral comporte à cet effet un moyen de traction 3 destiné à être attaché à ses extrémités 3a aux gouttières 1, 2. Les liens sont montés sur le coté extérieur des gouttières 1, 2 de manière à ce que leurs axes longitudinaux s'étendent dans un sens allant de l'arrière bas du maxillaire inférieur à l'avant haut du maxillaire supérieur, une fois le dispositif étant mis en place, ce qui est montré à la figure 1a.

Comme mentionné dans l'introduction, les liens latéraux subissent une traction dans le cas d'un mouvement vertical entre les maxillaires supérieur et inférieur. Par conséquent, ils sont normalement fabriqués en un matériau flexible, mais ne permettant pas une extension dans l'axe longitudinal des liens, de préférence également en plastique, par exemple en polyoxyméthylène (POM), un matériau fréquemment utilisé dans ce domaine, ou en métal comme par exemple du titane. Ceci vaut en particulier pour le moyen de traction 3 d'un lien latéral qui correspond à la partie intermédiaire d'un lien entre ses extrémités 3a et qui peut par exemple consister en une barre ou un cordon de raccordement.

Les extrémités 3a du moyen de traction 3 d'un lien latéral sont de préférence sphériques, mais elles pourraient également avoir une autre forme équivalente, par exemple semi-sphérique. De préférence, le moyen de traction 3 et les extrémités sphériques 3a d'un lien latéral sont venus d'une pièce, notamment dans la forme d'exécution ayant une barre de raccordement, au lieu d'être fixés l'un à l'autre après leur fabrication. Cette pièce d'attachement est disponible en plusieurs longueurs afin de pouvoir régler l'avancement du maxillaire inférieur, comme ceci apparaîtra plus clairement dans la suite.

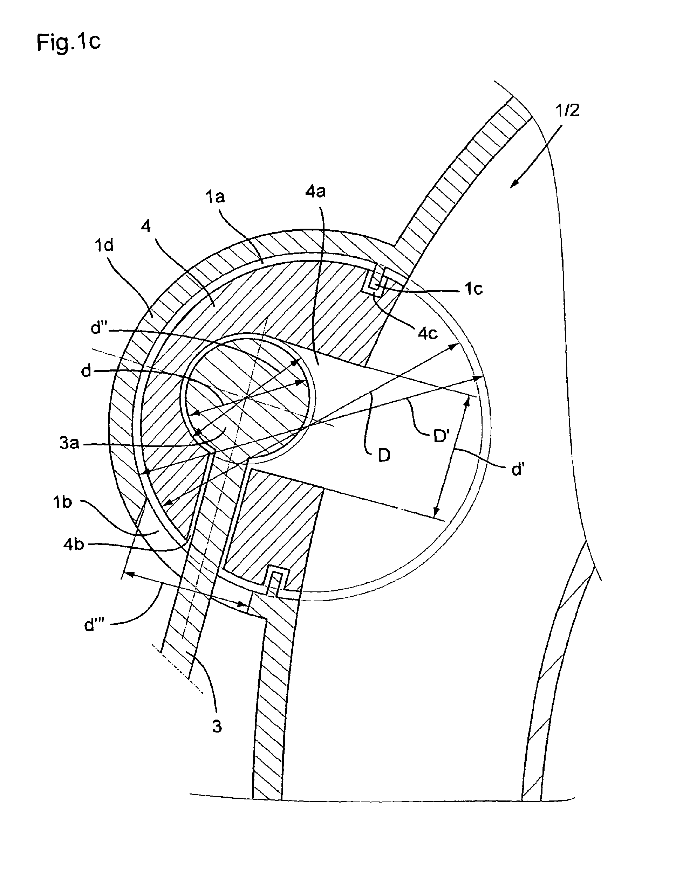

De plus, les liens latéraux d'un dispositif selon la présente invention comportent notamment des rotules 4 qui sont également illustrées aux figures 2a à 2c. Ces rotules 4 peuvent consister du même matériau que les moyens de traction 3 et ont chacune une ouverture 4a, 4b adaptée à recevoir une extrémité 3a d'un moyen de traction 3 du lien afin de connecter le moyen de traction 3 à chacune de ses extrémités 3a à une rotule 4. Simultanément, chaque gouttière 1, 2 comprend deux logements latéraux 1 d, 2d adaptés à héberger une rotule 4 connectée à un moyen de traction 3, ce qui est montré schématiquement aux figures 1b et 1c. Ainsi, les liens latéraux peuvent être connectés de façon amovible aux gouttières 1, 2 par l'intermédiaire d'un joint à rotule.

Cette conception générale permet qu'une ouverture de la bouche due à un abaissement du maxillaire inférieur pendant le sommeil provoque une propulsion du maxillaire inférieur en avant et une ouverture correspondante des voies respiratoires pharyngales.

Les logements latéraux 1 d, 2d d'une gouttière 1, 2 se situent sur son coté extérieur et comportent un trou 1 b, 2b qui sert à définir la direction de l'axe longitudinal du moyen de traction 3 qui y est logé. Ils comportent également une alvéole intérieure 1 a, 2a de forme sensiblement semi-sphérique, l'entrée sensiblement circulaire de cette alvéole intérieure 1 a, 2a voisinant l'arcade dentaire correspondante une fois le dispositif étant mis en place sur les arcades dentaires, comme ceci est représenté aux figures 1b et 1c.

Afin de fabriquer des gouttières 1, 2 avec ces logements latéraux 1 d, 2d, il suffit, comme indiqué ci-dessus, par exemple de placer aux endroits prévus pour les articulations des liens latéraux un négatif d'une rotule 4 en POM ou en un matériau désagrégeable sur une reproduction de l'arcade dentaire du patient obtenue à l'aide d'une empreinte individuelle. Ensuite, les gouttières sont thermoformées de manière conventionnelle, mais avec les négatifs en place, et chaque négatif peut être enlevé des logements latéraux 1 d, 2d ainsi formés une fois que les gouttières 1, 2 sont refroidies. Finalement, les logement latéraux 1 d, 2d peuvent être retravaillés par exemple par fraisage afin d'améliorer la forme des alvéoles intérieures 1 a, 2a et les trous 1 b, 2b peuvent être découpés aux emplacements désirés pour définir la direction de l'axe longitudinal du moyen de traction 3 logé dans l'alvéole intérieure 1a, 2a correspondante.

Les rotules 4 d'un lien latéral sont de forme sensiblement semi-sphérique et elles ont un diamètre extérieur D légèrement inférieur au diamètre intérieur D' des alvéoles intérieures 1 a, 2a des logements latéraux 1 d, 2d dans les gouttières dentaires 1, 2 afin de pouvoir être logées dans ces dernières.

De plus, comme indiqué dans la figure 1c, chaque rotule 4 comporte une rainure annulaire 4c autour de sa paroi extérieure entourant l'ouverture d'une cavité 4a décrite en détail ci-dessous, la rainure étant située environ au niveau du plan de la forme semi-sphérique de la rotule 4. Les alvéoles intérieures 1 a, 2a d'une gouttière 1, 2 hébergeant la rotule 4 disposent d'une languette annulaire 1 c, 2c adaptée à s'encliqueter dans la rainure annulaire 4c de la rotule 4, la languette étant de façon correspondante située environ au niveau de l'entrée sensiblement circulaire de l'alvéole intérieure 1 a, 2a. Ceci permet un mouvement rotatif de la rotule 4 dans le plan de la rainure annulaire 4c et de la languette annulaire 1 c, 2c. Evidemment il est possible de prévoir la disposition inverse, c'est-à-dire une languette annulaire autour de la paroi extérieure d'une rotule 4 et une rainure dans l'alvéole intérieure 1 a, 2a d'une gouttière 1, 2. Il n'est pas nécessaire que la languette soit annulaire; en pratiquant la rainure également uniquement sur une partie de la circonférence de la rotule voire du logement latéral, le mouvement rotatif pourrait être limité.

Pour décrire l'ouverture 4a, 4b d'une rotule 4 de façon détaillée, celle-ci comprend un passage oblong 4b adapté à recevoir une partie oblongue d'un moyen de traction 3 du lien, c'est-à-dire une partie de la barre ou du cordon de raccordement, et une cavité 4a de forme sensiblement semi-sphérique adaptée à recevoir une extrémité 3a d'un moyen de traction 3 du lien latéral, comme ceci est visible dans les figures 1c, 2a et 2c.

Le diamètre d des extrémités sphériques 3a d'un moyen de traction 3 est légèrement supérieur au diamètre d' de l'ouverture de la cavité 4a d'une rotule 4 au niveau de l'entrée sensiblement circulaire de cette cavité 4a et légèrement inférieur au diamètre intérieur d" de la cavité 4a. Les dimensions du passage oblong 4b correspondent normalement sensiblement aux dimensions du moyen de traction 3, de façon à permettre l'introduction du moyen de traction dans la rotule 4 et, une fois en place, un mouvement rotatif autour de l'axe longitudinal du moyen de traction 3. Cette conception permet un assemblage du moyen de traction 3 à la rotule 4 par emboîtement de l'extrémité 3a du moyen de traction 3 dans la cavité 4a de la rotule 4. Une saillie pourrait également être ajoutée au niveau du passage oblong 4b afin d'emboîter aussi le moyen de traction 3.

Finalement, le diamètre d"' du trou 1 b, 2b d'un logement latéral 1 d, 2d d'une gouttière 1, 2 est supérieur au diamètre d des extrémités sphériques 3a d'un moyen de traction 3 d'un lien latéral afin de permettre un assemblage du moyen de traction 3 à la rotule 4 à travers ce trou 1 b, 2b. L'emplacement de ce trou 1 b, 2b sur un logement latéral d'une gouttière 1, 2 est choisi en fonction de la position de ces logements sur la gouttière, de manière à ce que les trous définissent la direction de l'axe longitudinal du lien latéral et permettent une mobilité suffisante des liens ainsi que des gouttières 1, 2.

Les logements latéraux 1 d, 2d de la gouttière dentaire supérieure 1 se situent en effet sur son coté extérieur, fréquemment au niveau des canines, et les logements latéraux 1 d, 2d de la gouttière dentaire inférieure 2 sur son coté extérieur, au niveau des prémolaires. Les centres des trous 1 b, 2b sont normalement placés dans les logements latéraux 1 d, 2d de manière à définir l'axe longitudinal des moyens de traction 3 en ligne droite entre les centres des logements 1 d, 2d de chaque coté des gouttières 1, 2.

Pour être complet, il est à noter que l'emplacement des logements latéraux sur les gouttières 1, 2 pourrait être inversé, c'est-à-dire en avant sur le maxillaire inférieur et en arrière sur le maxillaire supérieur, afin d'obtenir un dispositif adapté à tirer le maxillaire inférieur en arrière. Ce type d'appareil peut évidemment être construit en utilisant les mêmes caractéristiques décrites ci-dessus et peut servir dans certaines applications qui visent à corriger une malformation au niveau des os faciaux et notamment du maxillaire inférieur.

En ce qui concerne le lien latéral à lui seul, il peut évidemment être fabriqué indépendamment des gouttières 1, 2 et inversement. Comme cela ressort de la description ci-dessus, un tel lien est destiné à être intégré dans un dispositif de propulsion mandibulaire comportant une gouttière dentaire supérieure 1 destinée à être placée sur l'arcade dentaire supérieure d'un patient et une gouttière dentaire inférieure 2 destinée à être placée sur l'arcade dentaire inférieure. Le lien est adapté à relier les deux gouttières 1, 2 de manière à avancer l'arcade dentaire inférieure en cas d'un mouvement vertical entre les deux arcades dentaires et comporte un moyen de traction 3 attaché à ses extrémités 3a aux gouttières 1, 2. En particulier, les extrémités 3a du moyen de traction 3 du lien sont adaptées à être logées de façon amovible dans une ouverture 4a, 4b d'une rotule 4 du lien latéral. Cette rotule 4 connectée à un moyen de traction 3 est adaptée à être hébergée de façon amovible dans un logement latéral 1 d, 2d d'une gouttière 1, 2. Ainsi, le lien latéral peut être connecté aux gouttières 1, 2 de façon amovible par l'intermédiaire d'un joint à rotule.

En ce qui concerne le procédé d'assemblage d'un dispositif de propulsion mandibulaire selon la présente invention, le procédé comporte les étapes suivantes :

D'abord, une extrémité 3a d'un moyen de traction 3 d'un lien latéral est passée à partir du coté extérieur d'une gouttière 1, 2 à travers le trou 1 b, 2b dans un logement latéral 1 d, 2d définissant la direction de l'axe longitudinal du moyen de traction 3 qui y est logé, de manière à ce que l'extrémité 3a soit accessible de l'autre coté du logement 1 d, 2d. Ensuite, une rotule 4 est emboîtée sur l'extrémité 3a du moyen de traction 3 du lien latéral passé par le trou 1 b, 2b d'un logement latéral 1 d, 2d. Cette rotule 4 est par la suite encliquetée dans l'alvéole intérieure 1 a, 2a du logement latéral 1 d, 2d d'une gouttière 1, 2, l'articulation étant ainsi achevée à une extrémité du lien latéral. Ces étapes sont répétées pour toutes les extrémités 3a des moyens de traction 3 des deux liens latéraux, de manière à ce que les liens latéraux soient tous connectés latéralement aux gouttières 1, 2 par l'intermédiaire d'un joint à rotule.

Il est à noter que les caractéristiques mentionnées ci-dessus aussi bien du dispositif que de son assemblage permettent un emboîtement amovible des trois composants importants du dispositif selon la présente invention l'un dans l'autre, notamment (de l'extrémité 3a) du moyen de traction 3 dans la rotule 4 et de cette dernière dans le logement latéral d'une gouttière 1, 2. De ce fait, le dispositif réalise, notamment à l'aide de la rotule 4, de façon simple et efficace une articulation correspondant à un joint à rotule. Cette disposition permet alors un léger mouvement latéral et un mouvement vertical, c'est-à-dire l'ouverture de la bouche, entre les maxillaires supérieur et inférieur tout en garantissant la propulsion en avant désirée du maxillaire inférieur en cas de mouvement vertical entre les deux. La possibilité de ces mouvements et le concept d'un joint à rotule rendent le dispositif plus résistant à la cassure. De plus, le logement latéral dans les gouttières forme sur le coté extérieur de l'appareil une protection pour le tissu de la bouche du patient vis-à-vis de l'articulation. En même temps, le logement latéral permet une sécurité accrue aussi dans le sens que, en cas de cassure d'un lien, il n'y a pas de risque pour le patient d'avaler une pièce du système car, contrairement à une fixation par clipsage, les extrémités des liens latéraux restent toujours logées à l'intérieur des gouttières et ne peuvent par conséquent pas se séparer de l'appareil. Du fait de l'emboîtement amovible, il est possible même pour l'utilisateur de facilement changer des liens latéraux voire des barres de raccordement et ainsi de régler aisément l'avancement du maxillaire inférieur en choisissant une barre de raccordement d'une longueur adéquate. Simultanément, ceci simplifie non seulement l'utilisation mais également la fabrication du dispositif. Ces avantages du dispositif selon la présente invention sont obtenus, tout en maintenant les modes de fabrication pour ce genre de produit ainsi que son coût, principalement grâce à la conception avantageuse du lien latéral.

## Revendications

1. Lien latéral, destiné à être intégré dans un dispositif de propulsion mandibulaire comportant une gouttière dentaire supérieure (1) destinée à être placée sur l'arcade dentaire supérieure d'un patient et une gouttière dentaire inférieure (2) destinée à être placée sur l'arcade dentaire inférieure, ledit lien étant adapté à relier les deux gouttières (1, 2) de manière à avancer ou reculer l'arcade dentaire inférieure lors d'un mouvement vertical entre les deux arcades dentaires en comportant un moyen de traction (3) attaché à ses extrémités (3a) aux gouttières (1, 2), **caractérisé par le fait que** les extrémités (3a) du moyen de traction (3) du lien sont adaptées à être logées de façon amovible dans une ouverture (4a, 4b) d'une rotule (4) du lien latéral, cette rotule (4) connectée à un moyen de traction (3) étant adaptée à être hébergée de façon amovible dans un logement latéral (1 d, 2d) d'une gouttière (1, 2), de manière à ce que le lien latéral puisse être connecté aux gouttières (1, 2) de façon amovible par l'intermédiaire d'un joint à rotule, et **par le fait que** ladite rotule (4) est de forme sensiblement semi-sphérique et que l'ouverture (4a, 4b) de ladite rotule (4) comprend un passage oblong (4b) adapté à recevoir une partie oblongue dudit moyen de traction (3) et une cavité (4a) de forme sensiblement semi-sphérique adaptée à recevoir une extrémité (3a) dudit moyen de traction (3).

2. Dispositif de propulsion mandibulaire comportant une gouttière dentaire supérieure (1) destinée à être placée sur l'arcade dentaire supérieure d'un patient et une gouttière dentaire inférieure (2) destinée à être placée sur l'arcade dentaire inférieure ainsi que deux liens latéraux adaptés à relier les deux gouttières (1, 2) de manière à avancer ou rétracter l'arcade dentaire inférieure lors d'un mouvement vertical entre les deux arcades dentaires, chaque lien latéral comportant un moyen de traction (3) attaché par ses extrémités (3a) aux gouttières (1, 2), **caractérisé par le fait que** chaque lien latéral est un lien latéral selon la revendication 1, qui comprend des rotules (4) ayant une ouverture (4a, 4b) adaptée à recevoir une extrémité (3a) d'un moyen de traction (3), **par le fait que** chaque gouttière (1, 2) comprend deux logements latéraux (1d, 2d) adaptés à héberger une rotule (4) connectée à un moyen de traction (3), de manière à ce que les liens latéraux soient connectés aux gouttières (1, 2) par l'intermédiaire d'un joint à rotule, et **par le fait que** chaque logement latéral (1d, 2d) d'une gouttière (1, 2) se situe sur son coté extérieur et comporte un trou (1 b, 2b) définissant la direction de l'axe longitudinal du moyen de traction (3) qui y est logé ainsi qu'une alvéole intérieure (1a, 2a) de forme sensiblement semi-sphérique, l'entrée sensiblement circulaire de cette alvéole intérieure (1a, 2a) voisinant l'arcade dentaire une fois le dispositif mis en place.

3. Dispositif selon la revendication précédente, **caractérisé par le fait que** chaque rotule (4) est de forme sensiblement semi-sphérique et que l'ouverture (4a, 4b) d'une rotule (4) comprend un passage oblong (4b) adapté à recevoir une partie oblongue d'un moyen de traction (3) et comprenant une cavité (4a) de forme sensiblement semi-sphérique adaptée à recevoir une extrémité (3a) d'un moyen de traction (3).

4. Dispositif selon les revendications 2 et 3, **caractérisé par le fait que** chaque rotule (4) comporte une rainure annulaire (4c) autour de sa paroi extérieure entourant l'ouverture de la cavité (4a), l'alvéole intérieure (1a, 2a) d'une gouttière (1, 2) hébergeant la rotule (4) disposant d'une languette annulaire (1c, 2c) adaptée à s'encliqueter dans la rainure annulaire (4c) de la rotule (4), de manière à permettre un mouvement rotatif de la rotule (4) dans le plan de la rainure annulaire (4c) et de la languette annulaire (1c, 2c).

5. Dispositif selon l'une des revendications 2 à 4, **caractérisé par le fait que** les extrémités (3a) d'un moyen de traction (3) sont sphériques.

6. Dispositif selon la revendication 5, **caractérisé par le fait que** le diamètre (d) des extrémités sphériques (3a) d'un moyen de traction (3) est supérieur au diamètre (d') de l'ouverture de la cavité (4a) d'une rotule (4) au niveau de l'entrée sensiblement circulaire de cette cavité (4a) et inférieur au diamètre intérieur (d") de la cavité (4a), de manière à permettre un assemblage du moyen de traction (3) à la rotule (4) par emboîtement.

7. Dispositif selon la revendication 6, **caractérisé par le fait que** le diamètre (d"') du trou (1b, 2b) d'un logement latéral (1d, 2d) d'une gouttière (1, 2) est supérieur au diamètre (d) des extrémités sphériques (3a) d'un moyen de traction (3), de manière à permettre un assemblage du moyen de traction (3) à la rotule (4) à travers ce trou (1 b, 2b).

8. Dispositif selon l'une des revendications 2 à 7, **caractérisé par le fait que** les logements latéraux (1d, 2d) de la gouttière dentaire supérieure (1) se situent sur son coté extérieur au niveau des canines et les logements latéraux (1d, 2d) de la gouttière dentaire inférieure (2) se situent sur son coté extérieur au niveau des prémolaires, les centres des trous (1 b, 2b) dans les logements latéraux (1d, 2d) étant placés de manière à définir la direction de l'axe longitudinal des moyens de traction (3) en ligne droite entre les centres des logements (1d, 2d) de chaque coté des gouttières (1, 2).

9. Dispositif selon l'une des revendications 2 à 8, **caractérisé par le fait qu'**un moyen de traction (3) consiste en une barre de raccordement ayant des extrémités sphériques (3a), le tout étant venu d'une pièce.

10. Procédé d'assemblage d'un dispositif de propulsion mandibulaire comportant une gouttière dentaire supérieure (1) destinée à être placée sur l'arcade dentaire supérieure d'un patient et une gouttière dentaire inférieure (2) destinée à être placée sur l'arcade dentaire inférieure ainsi que deux liens latéraux adaptés à relier les deux gouttières (1, 2) de manière à avancer ou reculer l'arcade dentaire inférieure lors d'un mouvement vertical entre les deux arcades dentaires, chaque lien latéral du dispositif comportant un moyen de traction (3) destiné à être attaché à ses extrémités (3a) aux gouttières (1, 2) et des rotules (4) ayant une ouverture (4a, 4b) adaptée à recevoir une extrémité (3a) d'un moyen de traction (3), chaque gouttière (1, 2) comprenant deux logements latéraux (1d, 2d) situés sur ses côtés extérieurs et adaptés à héberger une rotule (4) connectée à un moyen de traction (3), et chaque logement latéral (1d, 2d) comporte une alvéole intérieure (1a, 2a) ayant une entrée voisinant l'arcade dentaire une fois le dispositif mis en place, le procédé comportant les étapes de
- faire passer une extrémité (3a) d'un moyen de traction (3) d'un lien latéral à travers un trou (1 b, 2b) pratiqué dans le logement latéral (1d, 2d) définissant la direction de l'axe longitudinal du moyen de traction (3) qui y est logé,
- emboîter une rotule (4) sur l'extrémité (3a) du moyen de traction (3) du lien latéral passé à travers le trou (1b, 2b) d'un logement latéral (1d, 2d),
- encliqueter cette rotule (4) dans une alvéole intérieure (1a, 2a) du logement latéral (1d, 2d) et
- répéter ces étapes pour toutes les extrémités (3a) des moyens de traction (3) des deux liens latéraux,
de manière à ce que les liens latéraux soient connectés latéralement aux gouttières (1, 2) par l'intermédiaire de joints à rotule.

## Claims

1. Lateral link, to be incorporated into a mandibular protrusion device comprising an upper dental tray (1) to be placed onto the upper dental arch of a patient, and a lower dental tray (2) to be placed onto the lower dental arch, said link apt to link the two trays (1, 2) so as to advance or retract the lower dental arch during a vertical movement between the two dental arches, comprising a means of traction (3) attached via its ends (3a) to the trays (1, 2), **characterized by** the fact that the ends (3a) of the traction means (3) of the link are adapted to be detachably accommodated in an opening (4a,4b) of a ball pivot (4) of the lateral link, this ball pivot (4) connected with a means of traction (3), being adapted to be detachably lodged in a lateral seat (1d,2d) of a tray (1,2) in such a way that the lateral link may be detachably connected with the trays (1, 2) via a ball-and-socket joint, and by the fact that said ball pivot (4) is of substantially hemispherical shape and that the opening (4a,4b) of said ball pivot (4) comprises an oblong passage (4b) apt to receive an oblong segment of said means of traction (3) and a substantially hemispherical shaped cavity (4a) apt to receive one end (3a) of said means of traction (3).

2. Mandibular protrusion device comprising an upper dental tray (1) to be placed onto the upper dental arch of a patient, and a lower dental tray (2) to be placed onto the lower dental arch as well as two lateral links apt to link the two trays (1, 2) so as to advance or retract the lower dental arch during a vertical movement between the two dental arches, each lateral link comprising a means of traction (3) attached via its ends (3a) to the trays (1, 2), **characterized by** the fact that each lateral link is a lateral link according to claim 1, which comprises ball pivots (4) having an opening (4a, 4b) apt to receive one end (3a) of a means of traction (3), by the fact that each tray (1, 2) comprises two lateral seats (1d, 2d) apt to accommodate one ball pivot (4) connected with a means of traction (3), in such a way that the lateral links may be connected with the trays (1, 2) via a ball-and-socket joint, an by the fact that each lateral seat (1d, 2d) of a tray (1, 2) is located on its outer side, and comprises a hole (1b, 2b) defining the direction of the longitudinal axis of the means of traction (3) that is accommodated there, as well as an inner cup (1 a, 2a) of substantially hemispherical shape, the substantially circular entrance of said inner cup (1a, 2a) adjoining the dental arch when the device has been placed.

3. Device according to the preceding claim, **characterized by** the fact that each ball pivot (4) has an substantially hemispherical shape, and the opening (4a, 4b) of a ball pivot (4) comprises an oblong passage (4b) apt to receive an oblong segment of a means of traction (3) and comprising a cavity (4a) of substantially hemispherical shape apt to receive one end (3a) of a means of traction (3).

4. Device according to claims 2 and 3, **characterized by** the fact that each ball pivot (4) comprises an annular groove (4c) around its outside wall that circles the opening of the cavity (4a), the inner cup (1a, 2a) of a tray (1, 2) that accommodates the ball pivot (4) being provided with an annular tongue (1c, 2c) apt to snap fit into the annular groove (4c) of ball pivot (4) so as to allow a rotary movement of ball pivot (4) in the plane of the annular groove (4c) and annular tongue (1c, 2c).

5. Device according to one of claims 2 to 4, **characterized by** the fact that the ends (3a) of a means of traction (3) are spherical.

6. Device according to claim 5, **characterized by** the fact that the diameter (d) of the spherical ends (3a) of a means of traction (3) is larger than the diameter (d') of the opening of cavity (4a) of a ball pivot (4) at the level of the substantially circular entrance of this cavity (4a), and smaller than the inner diameter (d") of the cavity (4a), so as to allow a snap-fit assembly of the means of traction (3) with the ball pivot (4).

7. Device according to claim 6, **characterized by** the fact that the diameter (d"') of the hole (1b, 2b) of a lateral seat (1d, 2d) of a tray (1, 2) is larger than the diameter (d) of the spherical ends (3a) of a means of traction (3), so as to allow an assembly of the means of traction (3) with the ball pivot (4) through this hole (1b, 2b).

8. Device according to one of the claims 2 to 7, **characterized by** the fact that the lateral seats (1d, 2d) of the upper dental tray (1) are located on its outer side at the level of the canines, and the lateral seats (1d, 2d) of the lower dental tray (2) are located on its outer side at the level of the premolars, while the centers of the holes (1b, 2b) in the lateral seats (1 d, 2d) are positioned so as to define the direction of the longitudinal axis of the means of traction (3) in a straight line between the centers of the seats (1d, 2d) on each side of the trays (1, 2).

9. Device according to one of claims 2 to 8, **characterized in that** a means of traction (3) consists of a connecting rod having spherical ends (3a), and is integrally made as a single part.

10. Process of assembly of a mandibular protrusion device comprising an upper dental tray (1) to be placed onto the upper dental arch of a patient, and a lower dental tray (2) to be placed onto the lower dental arch, as well as two lateral links apt to link the two trays (1, 2) so as to advance or retract the lower dental arch during a vertical movement between the two dental arches, each lateral link of the device comprising a means of traction (3) to be attached with its ends (3a) to the trays (1, 2), as well as ball pivots (4) having an opening (4a, 4b) apt to receive one end (3a) of a means of traction (3), each tray (1, 2) comprising two lateral seats (1d, 2d) located on their outer sides and apt to accommodate one ball pivot (4) connected with a means of traction (3), and each lateral seat (1d, 2d) comprises an inner cup (1a, 2a) having an entrance adjoining the dental arch when the device has been placed, the process comprising the steps of
- sliding one end (3a) of a means of traction (3) of a lateral link through a hole (1b, 2b) made in the lateral seat (1d, 2d) defining the direction of the longitudinal axis of the means of traction (3) that is accommodated there,
- fitting a ball pivot (4) over the end (3a) of the means of traction (3) of the lateral link that has been pushed through the hole (1b, 2b) of a lateral seat (1d, 2d),
- snap fitting this ball pivot (4) into an inner cup (1a, 2a) of the lateral seat (1d, 2d), and
- repeating these steps for all ends (3a) of the means of traction (3) of the two lateral links,
in such a way that the lateral links be laterally connected with the trays (1, 2) via ball-and-socket joints.

## Patentansprüche

1. Seitliche Verbindung, die dazu bestimmt ist, Teil einer Vorrichtung zur Kieferprotrusion zu sein, welche eine Oberkieferschiene (1) aufweist, die dazu bestimmt ist, auf die obere Zahnreihe eines Patienten gesetzt zu werden, sowie eine Unterkieferschiene (2), die dazu bestimmt ist, auf die untere Zahnreihe gesetzt zu werden, wobei die Verbindung dazu geeignet ist, die beiden Zahnschienen (1, 2) derart zu verbinden, dass die untere Zahnreihe bei einer vertikalen Bewegung zwischen den beiden Zahnreihen nach vorn oder nach hinten verschoben wird, da sie ein Zugmittel (3) aufweist, das an seinen Enden (3a) an den Schienen (1, 2) befestigt ist, **dadurch gekennzeichnet, dass** die Enden (3a) des Zugmittels (3) der Verbindung dazu geeignet sind, auf bewegliche Weise in einer Öffnung (4a, 4b) eines Kugelelements (4) der seitlichen Verbindung angeordnet zu werden, wobei das Kugelelement (4), welches mit einem Zugmittel (3) in Verbindung steht, dazu geeignet ist, auf bewegliche Weise von einem seitlichen Lager (1d, 2d) einer Schiene (1, 2) aufgenommen zu werden, und zwar derart, dass die seitliche Verbindung über ein Kugelgelenk auf bewegliche Weise mit den Schienen (1, 2) verbunden werden kann, und **dadurch**, dass das Kugelelement (4) im Wesentlichen von halbkugelartiger Form ist und dass die Öffnung (4a, 4b) des Kugelelements (4) einen länglichen Durchlass (4b) umfasst, der dazu geeignet ist, einen länglichen Abschnitt des Zugmittels (3) aufzunehmen, sowie einen Hohlraum (4a), der im Wesentlichen von halbkugelartiger Form ist und der dazu geeignet ist, ein Ende (3a) des Zugmittels (3) aufzunehmen.

2. Vorrichtung zur Kieferprotrusion, welche eine Oberkieferschiene (1) aufweist, die dazu bestimmt ist, auf die obere Zahnreihe eines Patienten gesetzt zu werden, sowie eine Unterkieferschiene (2), die dazu bestimmt ist, auf die untere Zahnreihe gesetzt zu werden, sowie zwei seitliche Verbindungen, die dazu geeignet sind, die beiden Schienen (1, 2) derart zu verbinden, dass die untere Zahnreihe bei einer vertikalen Bewegung zwischen den beiden Zahnreihen nach vorn oder nach hinten verschoben wird, wobei jede der seitlichen Verbindungen ein Zugmittel (3) aufweist, das an seinen Enden (3a) an den Schienen (1, 2) befestigt ist, **dadurch gekennzeichnet, dass** es sich bei jeder der seitlichen Verbindungen um eine seitliche Verbindung gemäß dem Anspruch 1 handelt, welche Kugelelemente (4) aufweist, die eine Öffnung (4a, 4b) haben, welche dazu geeignet ist, ein Ende (3a) eines Zugmittels (3) aufzunehmen, sowie **dadurch**, dass jede Schiene (1, 2) zwei seitliche Lager (1d, 2d) umfasst, die dazu geeignet sind, ein Kugelelement (4), welches mit einem Zugmittel (3) in Verbindung steht, aufzunehmen, sodass die seitlichen Verbindungen mit den Schienen (1, 2) über ein Kugelgelenk verbunden sind, sowie **dadurch**, dass jedes der seitlichen Lager (1d, 2d) einer Schiene (1, 2) sich an deren Außenseite befindet und ein Loch (1b, 2b) aufweist, das die Ausrichtung der Längsachse des Zugmittels (3), welches darin angeordnet ist, festlegt, sowie einen innengelegenen Freiraum (1a, 2a) von im Wesentlichen halbkugelartiger Form, wobei der im Wesentlichen kreisförmige Eingang zu diesem innengelegenen Freiraum (1a, 2a) sich nach der Anbringung der Vorrichtung in unmittelbarer Nähe der Zahnreihe befindet.

3. Vorrichtung nach dem vorhergehenden Anspruch, **dadurch gekennzeichnet, dass** das Kugelelement (4) von im Wesentlichen halbkugelartiger Form ist und dass die Öffnung (4a, 4b) eines Kugelelements (4) einen länglichen Durchlass (4b) umfasst, der dazu geeignet ist, einen länglichen Abschnitt eines Zugmittels (3) aufzunehmen, und wobei es einen Hohlraum (4a) von im Wesentlichen halbkugelartiger Form umfasst, der dazu geeignet ist, ein Ende (3a) eines Zugmittels (3) aufzunehmen.

4. Vorrichtung gemäß den Ansprüchen 2 und 3, **dadurch gekennzeichnet, dass** jedes der Kugelelemente (4) eine Rille (4c) aufweist, die ringförmig um seine Außenwand verläuft, welche die Öffnung des Hohlraums (4a) umgibt, wobei der innengelegene Freiraum (1a, 2a) einer Schiene (1, 2), welcher das Kugelelement (4) aufnimmt, über einen ringförmigen Vorsprung (1c, 2c) verfügt, der dazu geeignet ist, in die ringförmige Rille (4c) des Kugelelements (4) derart einzurasten, dass das Kugelelement (4) in der Ebene der ringförmigen Rille (4c) und des ringförmigen Vorsprungs (1 c, 2c) eine Drehbewegung ausführen kann.

5. Vorrichtung nach einem der Ansprüche 2 bis 4, **dadurch gekennzeichnet, dass** die Enden (3a) eines Zugmittels (3) kugelförmig sind.

6. Vorrichtung nach dem Anspruch 5, **dadurch gekennzeichnet, dass** der Durchmesser (d) der kugelförmigen Enden (3a) eines Zugmittels (3) größer als der Durchmesser (d') der Öffnung des Hohlraums (4a) eines Kugelelements (4) im Bereich des im Wesentlichen kreisförmigen Eingangs zu diesem Hohlraum (4a) sowie kleiner als der Innendurchmesser (d") des Hohlraums (4a) ist, sodass das Zugmittel (3) durch einen Steckvorgang in das Kugelelement (4) eingefügt werden kann.

7. Vorrichtung nach dem Anspruch 6, **dadurch gekennzeichnet, dass** der Durchmesser (d"') des Lochs (1b, 2b) eines seitlichen Lagers (1d, 2d) einer Schiene (1, 2) größer als der Durchmesser (d) des kugelförmigen Enden (3a) eines Zugmittels (3) ist, sodass das Zugmittel (3) durch dieses Loch (1b, 2b) in das Kugelelement (4) eingefügt werden kann.

8. Vorrichtung nach einem der Ansprüche 2 bis 7, **dadurch gekennzeichnet, dass** sich die seitlichen Lager (1d, 2d) der Oberkieferschiene (1) auf deren Außenseite im Bereich der Schneidezähne befinden, während sich die seitlichen Lager (1d, 2d) der Unterkieferschiene (2) auf deren Außenseite im Bereich der prämolaren Zähne befinden, wobei die Mittelpunkte der Löcher (1b, 2b) in den seitlichen Lagern (1d, 2d) derart angeordnet sind, dass sie die Ausrichtung der Längsachse des Zugmittels (3) derart festlegen, dass sie zwischen den Mittelpunkten der Lager (1d, 2d) auf jeder Seite der Schienen (1, 2) eine Gerade bildet.

9. Vorrichtung nach einem der Ansprüche 2 bis 8, **dadurch gekennzeichnet, dass** ein Zugmittel (3) aus einer Verbindungsstange mit kugelförmigen Enden (3a) besteht, die aus einem einzigen Werkstück gearbeitet ist.

10. Verfahren zum Zusammenfügen einer Vorrichtung zur Kieferprotrusion, welche eine Oberkieferschiene (1) aufweist, die dazu bestimmt ist, auf die obere Zahnreihe eines Patienten gesetzt zu werden, sowie eine Unterkieferschiene (2), die dazu bestimmt ist, auf die untere Zahnreihe gesetzt zu werden, sowie zwei seitliche Verbindungen, die dazu geeignet sind, die beiden Schienen (1, 2) derart zu verbinden, dass die untere Zahnreihe bei einer vertikalen Bewegung zwischen den beiden Zahnreihen nach vorn oder nach hinten verschoben wird, wobei jede der seitlichen Verbindungen ein Zugmittel (3) aufweist, das dazu bestimmt ist, an seinen Enden (3a) an den Schienen (1, 2) befestigt zu werden, sowie Kugelelemente (4), die eine Öffnung (4a, 4b) haben, welche dazu geeignet ist, ein Ende (3a) eines Zugmittels (3) aufzunehmen, wobei jede Schiene (1, 2) zwei seitliche Lager (1d, 2d) umfasst, die sich an deren Außenseite befinden und dazu geeignet sind, ein Kugelelement (4), welches mit einem Zugmittel (3) in Verbindung steht, aufzunehmen, und wobei jedes der seitlichen Lager (1d, 2d) einen innengelegenen Freiraum (1a, 2a) aufweist, der einen Eingang hat, welcher sich nach der Anbringung der Vorrichtung in unmittelbarer Nähe der Zahnreihe befindet, wobei das Verfahren die folgenden Schritte umfasst
- Einführen eines Endes (3a) eines Zugmittels (3) einer seitlichen Verbindung durch ein Loch (1b, 2b), das im seitlichen Lager (1d, 2d) vorgesehen ist, welches die Ausrichtung der Längsachse des Zugmittels (3), das darin angeordnet ist, festlegt,
- Aufstecken eines Kugelelements (4) auf das Ende (3a) des Zugmittels (3) der seitlichen Verbindung, welche durch das Loch (1b, 2b) eines seitlichen Lagers (1d, 2d) geführt wurde,
- Einrasten des Kugelelements (4) in einem innengelegenen Freiraum (1a, 2a) des seitlichen Lagers (1d, 2d) und
- Wiederholen dieser Schritte für sämtliche Enden (3a) der Zugmittel (3) der beiden seitlichen Verbindungen,
sodass die seitlichen Verbindungen über ein Kugelgelenk seitlich mit den Schienen (1, 2) verbunden sind.
